# EUROPÄISCHE PATENTSCHRIFT

(11) **EP 0 709 104 B1**
(45) Veröffentlichungstag und Bekanntmachung des Hinweises auf die Patenterteilung: **11.03.1998**
(21) Anmeldenummer: 95116526.5
(22) Anmeldetag: 20.10.1995
(51) Int. Cl.: A61M 5/142, A61M 5/32, A61M 5/148

(54) **Injektionsvorrichtung**
Injection device
Dispositif d'injection

(30) Priorität: 28.10.1994 CH 3227/94
(43) Veröffentlichungstag der Anmeldung: 01.05.1996
(73) Patentinhaber: F. HOFFMANN-LA ROCHE AG, 4002 Basel (CH)
(72) Erfinder: Alex, Rainer, D-79189 Bad Krozingen (DE); Hadvary, Paul, CH-4105 Biel-Benken (CH); Tschirky, Hansjörg, CH-4107 Ettingen (CH)
(74) Vertreter: Buntz, Gerhard

(56) Entgegenhaltungen:
- EP-A- 0 272 530
- US-A- 4 393 870
- US-A- 4 725 265

## Beschreibung

Die Erfindung betrifft eine Injektionsvorrichtung mit einem Gehäuse und einem darin angeordneten Reservoir für die zu injizierende Flüssigkeit, einer mit dem Reservoir direkt kommunizierenden Kanüle, einer Einrichtung zum Einstechen der Kanüle durch einen Antrieb zur Erzeugung einer begrenzten linearen Bewegung und einer Pumpeinrichtung zum Entleeren des Reservoirinhalts durch die Kanüle.

Unter dem Begriff "Injektion" soll für den Zweck dieser Beschreibung sowohl das verhältnismässig schnelle Einspritzen (Bolus), als auch das langsamere, gelegentlich als Infusion oder Instillation bezeichnete Einbringen einer Flüssigkeit in den Körper verstanden werden.

Eine Injektionsvorrichtung der eingangs erwähnten Art ist beispielsweise aus EP-A-272 530 bekannt. In dieser Patentbeschreibung sind verschiedene Ausführungsformen einer solchen Injektionsvorrichtung beschrieben, bei denen unterschiedliche Arten von Reservoirs und unterschiedliche Pumpen verwendet werden. Allen dort gezeigten Ausführungsformen ist gemeinsam, dass ihr mechanischer Aufbau eine gewisse Mindestbauhöhe der gesamten Vorrichtung erfordert. Für Geräte, die von Patienten am Körper getragen werden, ist aber eine möglichst geringe Bauhöhe ein entscheidender Akzeptanzfaktor.

Ein weiterer Nachteil der bekannten Injektionsvorrichtungen, bei denen die Injektionskanüle in die Haut des Patienten eingeschossen wird, besteht darin, dass die Flüssigkeitsverbindung zwischen Kanüle und Reservoir problematisch ist. Zum einen muss diese Verbindung ziemlich kompliziert gestaltet werden, um der Relativbewegung zwischen der Kanüle und dem Reservoir Rechnung zu tragen. Dies bereitet Probleme für die Sterilität und die aseptische Abfüllung, v.a. von hitzeempfindlichen Präparaten. Ausserdem sind solche komplizierten Verbindungen störungsanfällig und ausserdem so teuer, dass sich die Vorrichtungen nicht als Wegwerfartikel eignen. Alternativ müsste im Fall einer festen Verbindung zwischen Reservoir und Kanüle zum Einschiessen das gesamte Reservoir mit Inhalt beschleunigt werden. Dies würde viel grössere Antriebskraft benötigen. Auch diese Lösung wäre unwirtschaftlich. In medizinischen Anwendungen sind häufig aber Einweg- oder Wegwerfartikel vorzuziehen. Zum zweiten ist es nicht trivial, das vorzeitige Austreten von Injektionslösung oder umgekehrt das Ansaugen einer geringen Menge Blut, Lymphe oder dergl. zu vermeiden.

Schliesslich gibt es bei den bekannten Injektionsvorrichtungen nach wie vor keine einfach und sicher funktionierende Sicherung gegen Verletzungen an der Kanüle nach Beendigung der Injektion und Abnahme der Vorrichtung vom Patienten.

Es besteht somit das Bedürfnis nach einer Injektionsvorrichtung die diese Nachteile nicht aufweist.

Zu diesem Zweck stellte sich die Aufgabe, einerseits die einzelnen Komponenten der Vorrichtung und andererseits das Zusammenwirken der Komponenten im Hinblick auf die erwünschte Verringerung der Bauhöhe, die Vereinfachung der mechanischen Funktion und die Vermeidung von Verletzungen an der Kanüle zu verbessern.

Erfindungsgemäss wird dies mit einer Injektionsvorrichtung gelöst, die sich durch die in den Patentansprüchen angegebenen Merkmale auszeichnet. Im Folgenden wird anhand der beiliegenden Zeichnungen ein Ausführungsbeispiel der Erfindung beschrieben. Es zeigen:
Fig. 1 eine schematische Schnittdarstellung einer Injektionsvorrichtung gemäss einer Ausführungsform der Erfindung
Fig. 2 eine schematische Schnittdarstellung einer alternativen Ausführungsform der Erfindung

Die anhand der Zeichnungen beschriebenen Ausführungsbeispiele sind Injektionsvorrichtungen, die vom Patienten auf sich getragen und von ihm selbst bedient werden können. Solche Injektionsvorrichtungen sind bekannt. Bei ihnen kommt es darauf an, die Injektionskanüle möglichst schmerzlos einzustechen, damit die bei vielen Patienten natürlich vorhandene Hemmung vermieden wird. Dieses Einstechen wird vorteilhaft mit einem erfindungsgemässen Antrieb vorgenommen.

Im Unterschied zu bekannten Vorrichtungen dieser Art wird jedoch zum Einstechen der Kanüle bei der vorliegenden Vorrichtung durch die Vorschubskraft des Federantriebs nicht die Kanüle relativ zum Gehäuse und zum Reservoir gegen die Haut des Patienten bewegt, sondern die Haut bewegt sich gegen die relativ zum Gehäuse und zum Reservoir feststehende Kanüle. Die Haut wird durch einen die Kanüle umgebenden Schutzring, der über den Gehäuseboden vorsteht, von der Kanüle ab und gleichzeitig unter Spannung gehalten. Zum Einstechen der Kanüle wird der Schutzring mittels Federkraft in das Gehäuse zurückgeschoben. Die dadurch frei werdende Haut bewegt sich gegen die Kanüle und wird durch deren Spitze durchstochen. Dass die Haut wider Erwarten bei diesem Vorgang eine für das Eindringen der Kanüle ausreichende Beschleunigung erfährt, ist überraschend. Ausserdem ist es überraschend, dass diese Art des Eindringens der Kanüle praktisch schmerzfrei erfolgt und zwar bis zu grösseren Eindringtiefen als dies bei bisher bekannten ähnlichen Vorrichtungen möglich war.

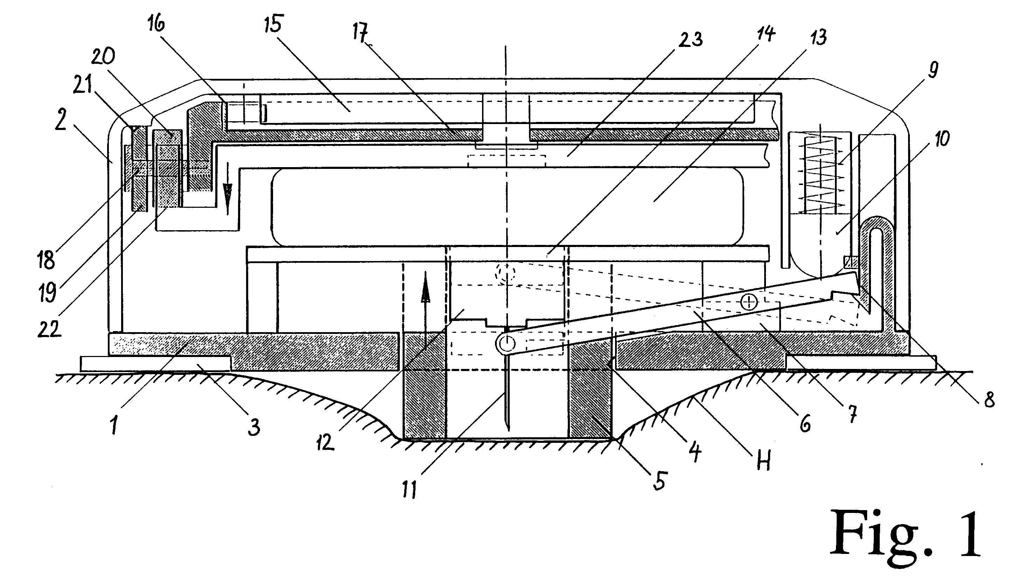

Fig. 1 zeigt eine derartige Injektionsvorrichtung im Schnitt mit einem Gehäuse aus einer scheibenförmigen Grundplatte 1 und einer relativ dazu drehbaren Kappe 2. Auf ihrer Unterseite ist die Grundplatte mit einer ringförmigen Klebeschicht 3 zur Befestigung der Vorrichtung auf der Haut H eines Patienten versehen. Die Grundplatte 1 besitzt eine zentrale konzentrische Oeffnung 4. In der Oeffnung ist eine zylindrische Schutzhülse 5 angeordnet, die axial beweglich ist zwischen der gezeigten vorgeschobenen Position und der gestrichelt angedeuteten zurückgezogenen Position, in der die untere Stirnseite des Schutzringes 5 mit der Unterseite der Grundplatte 1 in einer Ebene liegt.

Zum axialen Bewegen der Schutzhülse sind an dieser die einen Enden mehrerer, beispielsweise dreier, zweiarmiger oder gabelförmiger Hebel 6 verbunden, von denen einer in Fig. 1 gezeigt ist. Die Hebel 6 besitzen ihre Drehpunkte an Stützen 7, die mit der Kappe 2 starr verbunden sind. Die jeweils anderen Enden der Hebel erstrecken sich in der gezeigten Position in Verriegelungen 8. In der Ruheposition gespannte Spiralfedern 9 üben ihre Federkraft über kurze Kolben 10 auf die verriegelten Enden der Hebel aus.

Im Zentrum der Oeffnung ist eine Kanüle 11 angeordnet, die in einem Kanülenträger 12 sitzt, der seinerseits mit der Wand eines etwa flachzylindrisch geformten, flexiblen Reservoirs 13 zu einer Baueinheit verbunden ist. Das Reservoir ruht auf einem Unterbau aus einer Platte 14, die von den Stützen 7 gehalten wird. Reservoir 13, Kanülenträger 12 und Kanüle 11 sind somit relativ zum Gehäuse fest angeordnet. Die Kanüle 11 ragt dabei um die gewünschte Einstichtiefe über die Grundplatte hinaus. Die Schutzhülse ist so dimensioniert und angeordnet, dass sie in der Ruheposition die Kanüle 11 ausreichend überragt, um eine ungewollte Berührung mit der Kanülenspitze zu verhindern.

Die Vorrichtung wird mit der zum Schutz der Kanüle in der vorgeschobenen Position befindlichen Schutzhülse 5 mittels der Klebeschicht 3 an der gewünschten bzw. geeigneten Stelle auf der Haut H des Patienten befestigt. Durch die Schutzhülse 5 wird die Haut H in der gezeigten Weise von der Grundplatte weg gespannt.

Durch Drehen der Kappe 2 relativ zur Grundplatte 1 kann die Verriegelung 8 ausgelöst werden. Feder und Kolben drücken dann den frei gewordenen Hebelarm nach unten. Dadurch wird mit dem anderen Hebelarm die Schutzhülse in die gestrichelt gezeigte Position gebracht, d.h. in das Gehäuse zurückgezogen. Die dadurch frei werdende Haut H bewegt sich gegen die Grundplatte und wird dabei von der Kanülenspitze durchstochen, so dass die Kanüle 11 in die Haut eindringt.

Zur Entleerung des Inhalts des Reservoirs 13 durch die Kanüle 11 in das Hautgewebe des Patienten dient ein durch einen Elektromotor 15 mit Getriebe, beispielsweise ein Uhrwerk, über ein Zahngetriebe 16 bewegter Drehring 17, an dessen Aussenseite in gleichmässigen Winkelabständen drei auf gemeinsamen Achsen 18 gelagerte Rollenpaare 19, 20 angebracht sind. Die jeweils äusseren Rollen 19 laufen auf einer an der Innenseite der Kappe 2 geformten Schulter 21, während die jeweils inneren Rollen 20 auf keilförmigen Rampen 22 einer Druckplatte 23 laufen. Durch kontinuierliche Drehung des Drehringes 17 wird auf diese Weise die Druckplatte 23 langsam nach unten bewegt, so dass sie das Reservoir zusammenpresst und dessen Inhalt durch die Kanüle fördert.

Anstelle der auf den Rampen laufenden Rollen können auch einander gegenüberliegende gegenläufige Rampen am Drehring und an der Druckplatte vorgesehen werden. Diese Konstruktion ist einfacher, hat aber den Nachteil, dass wegen der Gleitreibung eine im Vergleich zur Rollreibung grössere Kraft erforderlich ist.

Eine andere Möglichkeit zur Entleerung des Reservoirs ist beispielsweise die Verwendung einer gasproduzierenden Zelle, mit der während einer bestimmten Zeitspanne ein Druck auf das Reservoir ausgeübt werden kann. Alternativ können auch andere Antriebe oder auch andere Arten von Reservoirs, wie sie im Stand der Technik bekannt sind, verwendet werden.

Die Entleerung des Reservoirs kann entweder kontinuierlich erfolgen oder entsprechend dem gemessenen Bedarf, der Konzentration des Wirkstoffes oder anderer Parameter gesteuert werden. Zu diesem Zweck kann mit der Kanüle 11 ein Miniatursensor, z.B. für Insulin, Glykose etc., verbunden sein, der mit in das Gewebe eingeführt wird. Alternativ kann ein Sensor mit einer zweiten in der Vorrichtung vorhandenen und in ähnlicher Weise eingeführten Kanüle oder an einer anderen, von der Vorrichtung entfernten Stelle an einen geeigneten Messort gebracht werden. Entsprechend ist der Motor mit einer Steuereinrichtung mit Empfangsmitteln für die Sensorsignale versehen.

Vor dem Wegnehmen der Vorrichtung vom Patienten können durch Weiterdrehen der Kappe die Hebelenden wieder in die Verriegelungen geführt werden, so dass die Schutzhülse wieder in ihre die Kanüle schützende Position vorgeschoben wird.

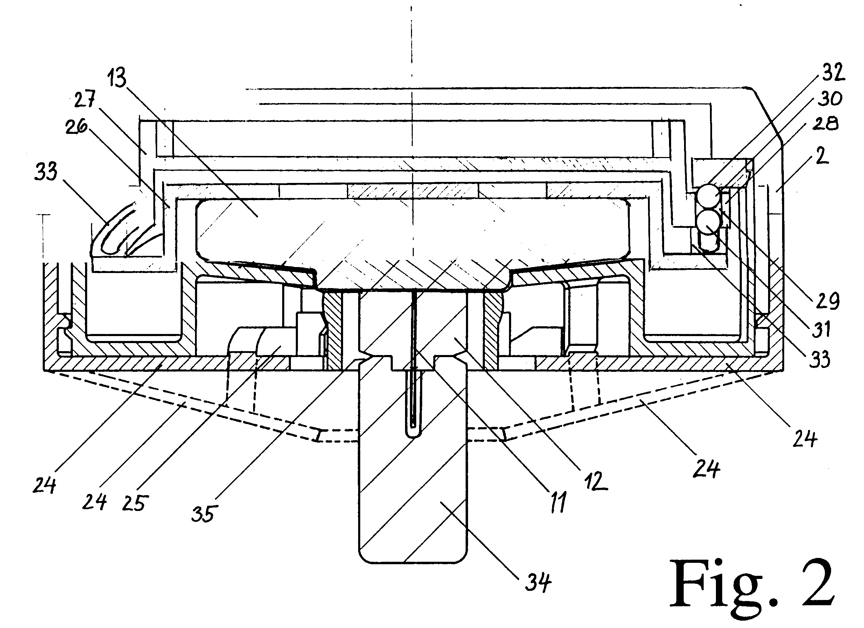

In Fig. 2 sind die der Fig. 1 entsprechenden Teile mit den gleichen Bezugszeichen versehen. Bei der Ausführungsform gemäss Fig. 2 ist kein Schutzring vorhanden, sondern der Boden 24 der Vorrichtung ist als Tellerfeder ausgebildet, die durch Druckelemente 25 in die gestrichelt gezeichnete vorgespannte Form gebracht wird, in der sie die Kanülenspitze überragt und damit deren ungewollte Berührung verhindert. Durch Auslösen der Druckelemente 25 schnappt der Boden 24 in seine flache Ruhelage, so dass sich die Haut des Patienten wie bei der vorstehend beschriebenen Ausführungsform gegen die Kanüle bewegt.

Auch bei dieser Ausführungsform kann der als Tellerfeder wirkende Boden der Vorrichtung vor deren Wegnahme vom Patienten mittels der Druckelemente wieder in die vorgespannte Position gebracht werden, in der er die Kanüle schützt.

Die Ausführungsform gemäss Fig. 2 zeigt ausserdem eine alternative Lösung für die Bewegung der Druckplatte 26 durch den Drehring 27. Der Drehring weist einen Flansch 28 mit drei in gleichmässigen Winkelabständen angeordneten Bohrungen 29 auf. In den Bohrungen befinden sich jeweils zwei Kugeln 30, 31, von denen die jeweils obere Kugel 30 sich an einer gehäusefesten Schulter 32 abstützt und die jeweils untere Kugel 31 auf einer Rampe 33 läuft. Die Schrägflächen der Rampen 33 sind zur Führung der Kugeln 31 mit rinnenförmigen Vertiefungen versehen. Durch kontinuierliche Drehung des Drehringes 27 wird wie bei der erstbeschriebenen Ausführungsform die Druckplatte 26 langsam nach unten bewegt, so dass sie das Reservoir zusammenpresst und dessen Inhalt durch die Kanüle fördert.

Die Ausführungsform gemäss Fig. 2 zeigt ferner eine Lösung für die Anforderung, dass der Inhalt des Reservoirs 13 und die Kanüle 11 steril sein müssen. Zu diesem Zweck ist ein Schutz 34 für die Kanüle 11 am Kanülenträger bzw. am Reservoir angeformt. Vorzugsweise erfolgt das Eingiessen der Kanüle 11 mit Kanülenträger und die Formung des Kanülenschutzes 34 gleichzeitig mit der Formung des Reservoirs 13 selbst. Ausserdem erfolgt die Formung des Reservoirs, seine Füllung mit der Wirkstofflösung, das Einsetzen von Kanüle und Kanülenträger, und das Verschliessen unter aseptischen Bedingungen vorzugsweise in einem unter dem Begriff Blow-Fill-Seal-Technik bekannten kontinuierlichen Arbeitsgang. Vor dem Aufsetzen der Vorrichtung auf die Haut des Patienten wird der Schutz von der Kanüle 11 entfernt. Zu diesem Zweck ist eine Sollbruchstelle 35 zwischen Schutz 34 und Kanülenträger 12 vorgesehen.

## Patentansprüche

1. Injektionsvorrichtung mit einem Gehäuse mit einer Kontaktfläche (1) zum Aufsetzen auf die Haut eines Patienten und einer im Gehäuse angeordneten Kanüle (11) zum Einstechen und Einbringen einer Injektionsflüssigkeit in die Haut, dadurch gekennzeichnet, dass die Kanüle relativ zum Gehäuse fest angeordnet und von einem Schutzelement (5) umgeben ist, das axial bewegbar ist zwischen einer ersten Endposition, in der das Schutzelement die Kanülenspitze überragt, und einer zweiten, in das Gehäuse zurückgezogenen Endposition, in der die Kanüle über die Kontaktfläche vorsteht, und dass ein Antrieb (6, 9, 10) zum axialen Bewegen des Schutzelements vorhanden ist.

2. Injektionsvorrichtung nach Anspruch 1, dadurch gekennzeichnet, dass im Gehäuse ein mit der Kanüle kommunizierendes Reservoir (13) für die Injektionsflüssigkeit und eine Pumpe zum Entleeren des Reservoirinhalts durch die Kanüle angeordnet ist.

3. Injektionsvorrichtung nach Anspruch 1, dadurch gekennzeichnet, dass Anschlussmittel zum Anbringen einer Zuleitung für die Injektionsflüssigkeit vorhanden ist.

4. Injektionsvorrichtung nach einem der vorangehenden Ansprüche, dadurch gekennzeichnet, dass das Schutzelement eine durch einen Federantrieb (9) bewegbare zylindrische Hülse (5) ist, die in ihrer einen Endposition aus dem Gehäuse herausragt und sich in ihrer anderen Endposition vollständig im Inneren des Gehäuses befindet.

5. Injektionsvorrichtung nach einem der Ansprüche 1- 3, dadurch gekennzeichnet, dass das Schutzelement aus dem als Tellerfeder ausgebildeten Gehäuseboden (24) besteht, der mittels Druckelementen(25) in eine gespannte Form gebracht wird, in der er die Kanülenspitze überragt.

6. Injektionsvorrichtung nach einem der vorangehenden Ansprüche, dadurch gekennzeichnet, dass die Kontaktfläche (1, 24) und ggf. das Schutzelement (5, 24) mit einer Klebeschicht (3) versehen sind.

7. Injektionsvorrichtung nach einem der Ansprüche 1- 3, dadurch gekennzeichnet, dass das Reservoir ein flachzylindrischer, flexibler Behälter ist

8. Injektionsvorrichtung nach Anspruch 7, dadurch gekennzeichnet, dass die Kanüle mit dem Reservoir und einer Schutzhülle eine Baueinheit bildet.

9. Injektionsvorrichtung nach Anspruch 8, dadurch gekennzeichnet, dass die aus Reservoir, Kanüle und Schutzhülle bestehende Baueinheit nach dem sog. Blow-Fill-Seal-Verfahren hergestellt ist.

10. Injektionsvorrichtung nach Anspruch 1, gekennzeichnet durch einen Sensor.

11. Injektionsvorrichtung nach Anspruch 8, dadurch gekennzeichnet, dass sie mehrfach verwendbar und die genannte Baueinheit auswechselbar ist.

## Claims

1. An injection device comprising a casing having a contact surface (1) for applying to a patient's skin and containing a cannula (11) disposed in the casing and adapted to pierce the skin and introduce an injection liquid into the skin, characterised in that the cannula is fixed relatively to the casing and is surrounded by a protective element (31) axially movable between a first end position in which the protective element projects beyond the tip of the cannula, and a second end position retracted into the casing, in which second end position the cannula projects beyond the contact surface, and in that a drive (6, 9, 10) is provided for axial movement of the protective element.

2. An injection device according to claim 1, characterised in that a reservoir (13) for the injection liquid is disposed in the casing in communicating relationship with the cannula and a pump is provided for emptying the reservoir contents through the cannula.

3. An injection device according to claim 1, characterised in that connecting means are provided for attaching a feed line for the injection liquid.

4. An injection device according to any one of the preceding claims, characterised in that the protective element is a cylindrical sleeve (5) which is movable by a spring drive (9) and which in one end position projects from the casing and in the other end position is located completely inside the casing.

5. An injection device according to any one of claims 1 to 3, characterised in that the protective element consists of the casing base (24) in the form of a cup spring which is brought by pressure elements (25) into a prestressed shape in which it projects beyond the cannula tip.

6. An injection device according to any one of the preceding claims, characterised in that the contact surface (1, 24) and optionally the protective element (5, 24) are provided with an adhesive layer (3).

7. An injection device according to any one of claims 1 to 3, characterised in that the reservoir is a flat cylindrical flexible container.

8. An injection device according to claim 7, characterised in that the cannula forms an integral unit with the reservoir and a protective sheath.

9. An injection device according to claim 8, characterised in that the unit consisting of the reservoir, cannula and protective sheath is produced by the blow-fill-seal process.

10. An injection device according to claim 1, characterised by a sensor.

11. An injection device according to claim 8, characterised in that it is re-usable and the said integral unit is replaceable.

## Revendications

1. Dispositif d'injection comportant un boîtier pourvu d'une surface de contact (1) destinée à être appliquée sur la peau d'un patient, et une canule (11) disposée dans le boîtier et destinée à perforer la peau et insérer un liquide d'injection dans la peau, caractérisé en ce que la canule est montée fixée par rapport au boîtier et est entourée par un élément de protection (5), qui est déplacable axialement entre une première position d'extrémité, dans laquelle l'élément de protection fait saillie au-delà de la pointe de la canule, et une seconde position d'extrémité, qui est rétractée dans le boîtier et dans laquelle la canule fait saillie au-dessus de la surface de contact, et qu'il est prévu un dispositif d'entraînement (6, 9, 10) servant à déplacer axialement l'élément de protection.

2. Dispositif d'injection selon la revendication 1, caractérisé en ce qu'un réservoir (13), qui communique avec la canule et est prévu pour le liquide d'injection, et une pompe servant à vider le contenu du réservoir au moyen de la canule sont disposés dans le boîtier.

3. Dispositif d'injection selon la revendication 1, caractérisé en ce que des moyens de raccordement sont prévus pour le montage d'une canalisation d'alimentation pour le liquide d'injection.

4. Dispositif d'injection selon l'une des revendications précédentes, caractérisé en ce que l'élément de connexion est une douille cylindrique (5) qui est déplacable à l'aide d'un dispositif d'entraînement à ressort (9) et qui, dans l'une de ses positions d'extrémité, ressort du boîtier et, dans son autre position d'extrémité, est située complètement à l'intérieur du boîtier.

5. Dispositif d'injection selon l'une des revendications 1-3, caractérisé en ce que l'élément de protection est constitué par le fond (24) du boîtier, qui est agencé sous la forme d'un ressort Belleville et est amené, à l'aide d'éléments de compression (25), dans un état bandé, dans lequel il fait saillie au-delà de la pointe de la canule.

6. Dispositif d'injection selon l'une des revendications précédentes, caractérisé en ce que la surface de contact (1, 24) et éventuellement l'élément de protection (5, 24) sont pourvus d'une couche adhésive (3).

7. Dispositif d'injection selon l'une des revendications 1-3, caractérisé en ce que le réservoir est un réservoir cylindrique plat flexible.

8. Dispositif d'injection selon la revendication 7, caractérisé en ce que la canule forme une unité de construction avec le réservoir et une douille de protection.

9. Dispositif d'injection selon la revendication 8, caractérisé en ce que l'unité de construction constituée par le réservoir, la canule et la douille de protection est fabriquée au moyen du procédé dit Blow-Fill-Seal.

10. Dispositif d'injection selon la revendication 1, caractérisé par un capteur.

11. Dispositif d'injection selon la revendication 8, caractérisé en ce qu'il est utilisable à plusieurs reprises et que ladite unité de construction est remplaçable.
